(19) Europäisches Patentamt
European Patent Office
Office européen des brevets

(11) Veröffentlichungsnummer: **0 129 252**
**B1**

(12) # EUROPÄISCHE PATENTSCHRIFT

(45) Veröffentlichungstag der Patentschrift: **08.08.90**

(21) Anmeldenummer: **84107062.6**

(22) Anmeldetag: **20.06.84**

(51) Int. Cl.$^5$: **C 07 D 311/72,** C 07 C 41/02, C 07 D 303/18, C 07 D 303/14, C 07 C 43/205

(54) **Verfahren zur Herstellung von optisch aktiven Hydrochinonderivaten sowie von d-alpha-Tocopherol.**

(30) Priorität: **21.06.83 CH 3391/83**

(43) Veröffentlichungstag der Anmeldung:
**27.12.84 Patentblatt 84/52**

(45) Bekanntmachung des Hinweises auf die Patenterteilung:
**08.08.90 Patentblatt 90/32**

(84) Benannte Vertragsstaaten:
**AT BE CH DE FR GB IT LI NL**

(56) Entgegenhaltungen:
**EP-A-0 058 945**
**EP-A-0 107 806**

**THE JOURNAL OF ORGANIC CHEMISTRY, Band 46, Nr. 12, 5. Juni 1981, Seiten 2445-2450; N. COHEN et al.: "Studies on the total synthesis of (2R, 4'R, 8'R)-alpha-tocopherol (Vitamin E). Stereospecific cyclizations leading to optically active chromans"**

**Houben-Weyl: Methoden der organischen Chemie, VI/3, Teil 3, Seiten 442-443**

(73) Patentinhaber: **F. HOFFMANN-LA ROCHE AG**
**Postfach 3255**
**CH-4002 Basel (CH)**

(72) Erfinder: **Barner, Richard, Dr.**
**Traubenweg 16**
**CH-4108 Witterswil (CH)**
Erfinder: **Hübscher, Josef**
**Spausel 1080**
**CH-5703 Seon (CH)**

(74) Vertreter: **Cottong, Norbert A. et al**
**Grenzacherstrasse 124 Postfach 3255**
**CH-4002 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 129 252 B1

**Beschreibung**

Die vorliegende Erfindung betrifft ein neues Verfahren zur Herstellung von Hydrochinonderivaten, welche als Zwischenprodukte für die Herstellung von d-α-Tocopherol (natürliches Vitamin E) geeignet sind, sowie gewünschtenfalls deren Überführung in d-α-Tocopherol selbst.

Es sind bereits einige Verfahren zur Herstellung von natürlichem Vitamin E bekannt, welche jedoch technisch nur von begrenztem Interesse sind, so dass natürliches Vitamin E bis heute nahezu ausschliesslich aus natürlichen Quellen extrahiert wird, siehe z.B. auch EP—A 58 945, wo von natürlichem Phytol ausgegangen wird, und immer noch ≥ 9 Stufen involviert sind.

Es bestand somit ein Bedürfnis nach einem technisch realisierbaren Verfahren, gemäss welchem natürliches Vitamin E in guter Ausbeute und mit hoher optischer Reinheit erhalten werden kann. Dies ist nunmehr mittels des erfindungsgemässen Verfahrens möglich.

Dieses Verfahren ist dadurch gekennzeichnet, dass man eine Verbindung der allgemeinen Formel

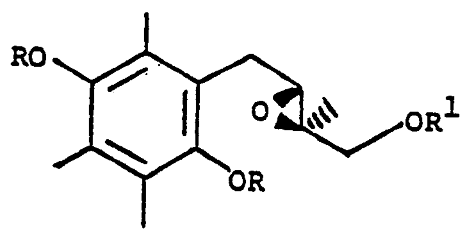

I

worin R eine Aetherschutzgruppe und $R^1$ Wasserstoff oder eine Aetherschutzgruppe darstellen, mit Wasserstoff in Gegenwart von Raney-Nickel oder, falls $R^1$ eine Aetherschutzgruppe darstellt, mit Lithiumaluminiumhydrid, reduziert, dass man die so erhaltene Verbindung der allgemeinen Formel

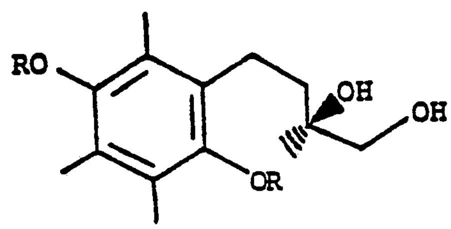

II

worin R die obige Bedeutung hat, in das Epoxid überführt, und dass man das so erhaltene Epoxid der allgemeinen Formel

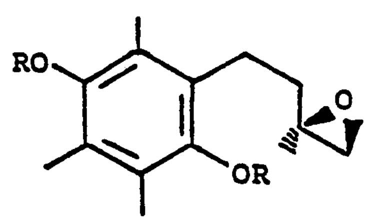

III

worin R die obige Bedeutung hat, mit einer Grignard-Verbindung der allgemeinen Formel

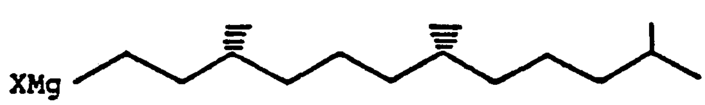

IV

worin X Brom oder Chlor darstellt, umsetzt zur Bildung der entsprechenden Verbindung der allgemeinen Formel

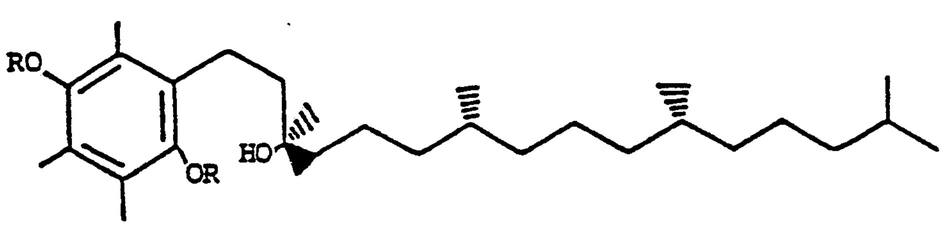

V

worin R die obige Bedeutung hat, in d-α-Tocopherol der Formel welche Verbindung man gewunschtenfalls

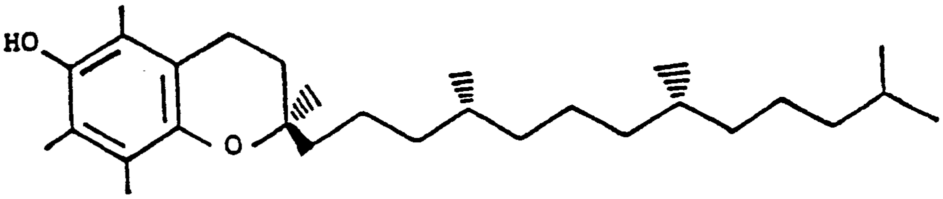

VI.

überführt.

Der Ausdruck "Aetherschutzgruppe" bedeutet im Rahmen der vorliegenden Erfindung sowohl durch Hydrolyse spaltbare Gruppen wie etwa die Silylgruppe oder Alkoxymethyl gruppen, beispielsweise die Methoxymethylgruppe, oder auch die Tetrahydropyranylgruppe, als auch oxidativ spaltbare Gruppen wie etwa $C_1$—$C_6$ Alkyläthergruppen. Weiterhin bedeutet das Zeichen "", dass sich der entsprechende Rest oberhalb der Molekülebene befindet, während das Zeichen "" bedeutet, dass sich der entsprechende Rest unterhalb der Molekülebene befindet.

Die Reduktion einer Verbindung der Formel I mit Wasserstoff in Gegenwart von Raney-Nickel kann unabhängig davon erfolgen, ob $R^1$ Wasserstoff oder eine Aetherschutzgruppe darstellt. Die Reduktion erfolgt zweckmässig in einen wässrigen System. Als zusätzliche Lösungsmittel kommen hier noch in Frage mit Wasser mischbare organische Lösungsmittel, insbesondere niedere Alkohole, wie z.B. Methanol, Aethanol oder Propanol usw. oder Aether wie Tetrahydrofuran und Dioxan oder auch Ketone wie etwa Aceton und dergleichen. Die Reduktion erfolgt weiterhin zweckmässig in neutralem bis schwach alkalischem Bereich, insbesondere im pH-Bereich von etwa 7—10 und bei einer Temperatur von etwa 70°C bis etwa 100°C, vorzugsweise bei Rückflusstemperatur des Reaktionsgemisches. Besonders bevorzugt ist ein Lösungsmittelgemisch, dessen Siedepunkt möglichst nahe bei 100°C liegt. Falls $R^1$ in einer Verbindung der Formel I eine Aetherschutzgruppe darstellt, kann die Reduktion auch mittels Lithiumaluminiumhydrid (Li Al $H_4$) durchgeführt werden. Dies erfolgt zweckmässig unter den für Reduktionen mittels Li Al $H_4$ üblichen Bedingungen, beispielsweise in einem Aether bei Raumtemperatur.

Die Ueberführung einer Verbindung der Formel II in ein Epoxid der Formel III kann in an sich bekannter Weise durchgeführt werden. Zu diesem Zweck wird in einer Verbindung der Formel II zunächst die primäre Hydroxygruppe in eine Abgangsgruppe übergeführt, z.B. in ein Halogenid (als Halogen kommen hier Chlor, Brom und Jod in Frage) oder in einen Sulfonsäureester (z.B. Tosylat oder Mesylat) und dergleichen. Dies kann in an sich bekannter Weise erfolgen. Anschliessend wird die so erhaltene Verbindung mit einer Base behandelt. Als Basen eignen sich sowohl anorganische als auch organische Basen, vorzugsweise jedoch anorganische Basen wie insbesondere Natriumhydroxid oder Kaliumhydroxid und dergleichen.

Die Umsetzung eines Epoxids der Formel III mit einer Grignard-Verbindung der Formel IV kann in an sich bekannter Weise durchgeführt werden. Bevorzugt ist jedoch die Umsetzung in Gegenwart von Kupfer (I oder II)-Katalysatoren, insbesondere Kupfer(I)-n-Propylacetylid oder einem Kupfer(I)-halogenid-dimethylsulfid-Komplex. Als Lösungsmittel eignen sich für diese Umsetzung alle bei Grignard Reaktionen üblicherweise in Frage kommenden Lösungsmittel.

Die Verbindungen der allgemeinen Formel V sind bekannt und können in bekannter Weise in d-α-Tocopherol übergeführt werden. Dies kann beispielsweise, falls der Rest R durch Hydrolyse abspaltbar ist, in einfacher Weise durch Behandlung mit einer Säure erfolgen. Falls der Rest R oxidativ abspaltbar ist, erfolgt die Ueberführung in einfacher Weise durch Behandlung mit z.B. Cerammoniumnitrat $[(Ce(NH_4)_2(NO_3)_6]$ und anschliessende reduktive Cyclisierung des erhaltenen Chinons.

Die in dem erfindungsgemässen Verfahren als Ausgangsmaterial verwendeten Verbindungen der Formel I sind neu. Sie können jedoch in an sich bekannter Weise nach folgendem Schema hergestellt werden. In den Formeln VII und IX hat R die vorhergende Bedeutung und X bedeutet Chlor oder Brom.

VII

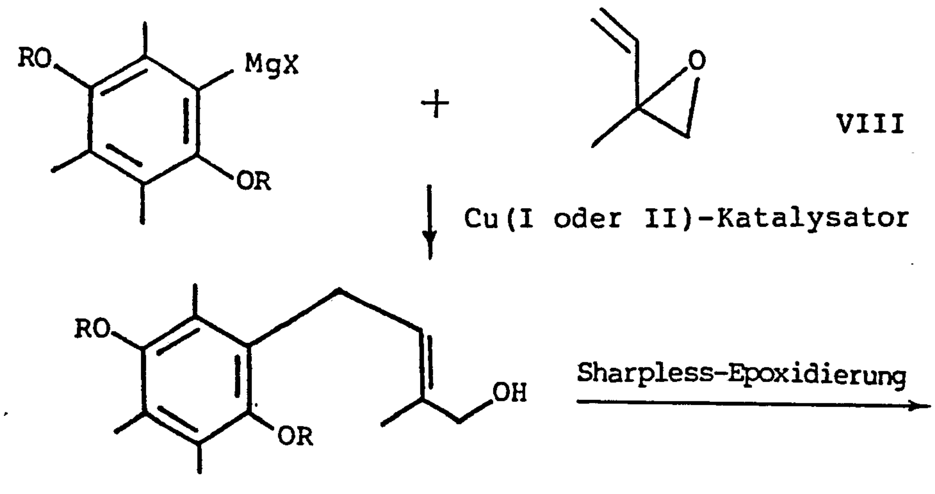


VIII

IX

I

Die Umsetzung einer Verbindung der Formel VII mit dem Isoprenoxid der Formel VIII kann unter den für eine Grignard-Reaktion üblichen Bedingungen, jedoch unter zusätzlicher Verwendung eines Kupfer (I oder II)-Katalysators durchgeführt werden. Besonders geeignet ist in diesem Fall Lithiumtetrachlorokuprat. Die anschliessende Sharpless-Epoxidierung ist eine an sich bekannte Reaktion und kann unter den üblichen Bedingungen durchgeführt werden.

Die Verbindungen der Formeln VII und VIII sich bekannt oder Analoge bekannter Verbindungen, welche leicht in zur Herstellung der bekannten Verbindungen analoger Weise hergestellt werden können. Die Verbindungen der Formel IX sind jedoch neu und ebenfalls Gegenstand der vorliegenden Erfindung.

## Beispiel 1

185 mg (2R,3R)-4-(2',5'-Dimethoxy-3',4',6'-trimethylphenyl)-2,3-epoxy-2-methylbutanol wurden in 5 ml Methanol gelöst und anschliessend mit 5 ml Wasser verdünnt. Hierauf wurde Raney-Nickel zugegeben und das Gemisch 2 Stunden unter Wasserstoff am Rückfluss erhitzt. Nach beendeter Wasserstoffaufnahme wurde filtriert, mit Methanol und Methylenchlorid gewaschen und am Rotationsverdampfer eingeengt. Zurückbleibendes Wasser wurde durch Zugabe von Methylenchlorid azeotrop abdestilliert. Das erhaltene Oel wurde aus Hexan/Aether umkristallisiert und man erhielt 161 mg 55%iges (S)-4-(2',5'-Dimethoxy-3',4',6'-trimethylphenyl)-2-methyl-1,2-butandiol (48% Ausbeute; GC-Analyse des Acetonids). Daten der Reinsubstanz: Smp. 86—87°C. $[\alpha]_D^{20}$ +2,55° (c = 5,3% in $CHCl_3$).

Das als Ausgangsmaterial verwendete (2R,3R)-4-(2',5'-Dimethoxy-3',4',6'-trimethylphenyl)-2,3-epoxy-2-methylbutanol kann wie folgt hergestellt werden:

a) 26,7 mMol 2,5-Dimethoxy-3,4,6-trimethylbenzylmagnesiumbromid wurden bei −78°C mit 3,6 g Lithiumtetrachlorokuprat ($Li_2CuCl_4$) als 0,1 molare Lösung in Tetrahydrofuran versetzt. Anschliessend wurden 2,2 g (26 mMol) Isoprenoxid zugegeben. Hierauf wurde das Gemisch 1 Stunde bei −78°C und 3—4 Stunden bei Raumtemperatur gerührt. Bie 0°C wurden dann 10 ml gesättigtes Ammoniumchlorid zugegeben und das Gemisch mit Aether extrahiert. Nach dem Trocknen der Aetherextrakte über Natriumsulfat, Einengen und Kristallisation aus Aethyläther bei −20°C erhielt man 4,7 g (75%) 4-(2',5'-Dimethoxy-3',4',6'-trimethylphenyl)-2-methyl-2-butenol mit einem Schmelzpunkt von 85—86°C.

b) 0,594 ml Titantetraisopropoxid wurden in 10 ml trockenem Methylenchlorid gelöst. Hierauf wurden bei −20°C 524 mg Dibutyl-D-tartrat zugetropft und das Gemisch 10 Minuten bei −20°C stehen gelassen. Dann wurden 197 mg 4-(2',5'-Dimethoxy-3',4',6'-trimethylphenyl)-2-methyl-2-butenol zugegeben und anschliessend noch 180 mg tert.Butylhydroperoxid (80%) zugetropft (als Lösung in 0,5 ml Methylenchlorid). Die so erhaltene gelbe Lösung wurde 4 bis 5 Tage bei −20°C stehen gelassen, dann mit 5 ml 1N Natronlauge versetzt, auf Raumtemperatur erwärmen gelassen und 1 Stunde gerührt. Die Phasen wurden dann getrennt, die Wasserphase zweimal mit Methylenchlorid gewaschen, die organischen Phasen über Natriumsulfat getrocknet und eingeengt. Das erhaltene farblose Oel wurde in 20 ml Aethyläther gelöst und mit 5 ml 1N Natronlauge 1 Stude gerührt. Die Phasen wurden wiederum getrennt, die Wasserphase zweimal mit Aethyläther gewaschen, die organischen Phasen über Natriumsulfat getrocknet und eingeengt. Man erhielt 188 mg (98%) (2R,3R)-4-(2',5'-Dimethoxy-3',4',6'-trimethylphenyl)-2,3-epoxy-2-methylbutanol.

$[\alpha]_D^{20}$ +18,2° (c = 2% in Chloroform).

## Beispiel 2

1,10 g (2R,3R)-4-(2',5'-Dimethoxy-3',4',6'-trimethylphenyl)-2,3-epoxy-2-methylbutanol wurden in 2 ml Pyridin gelöst. Dann wurden 0,5 ml Trimethylchlorsilan zugesetzt und das Gemisch 1 Stunde bei Raumtemperatur stehen gelassen. Hierauf wurde Natriumbicarbonatlösung zugegeben und das Gemisch mit Toluol extrahiert. Die organischen Phasen wurden über Natriumsulfat getrocknet und eingeengt. Das so erhaltene (2R,3R)-4-(2',5'-Dimethoxy-3',4',6'-trimethylphenyl)-2,3-epoxy-2-methyl-1-(trimethylsilyloxy)-butan wurde in 10 ml Aether gelöst, mit 40 mg Lithiumaluminiumhydrid versetzt und 16 Stunden bei Raumtemperatur gerührt. Dann wurde Ammoniumhydrogendifluorid-Lösung zugegeben und anschliessend mit Essigester extrahiert. Die organischen Phasen wurden über Natriumsulfat getrocknet, eingeengt und im Hochvakuum weiter getrocknet. Man erhielt dabei 804 mg (73%) (S)-4-(2',5'-Dimethoxy-3',4',6'-trimethylphenyl)-2-methyl-1,2-butandiol. Smp. 86—87°C. $[\alpha]_D^{20}$ +2,53° (c = 5,3% in $CHCl_3$).

Das als Ausgangsmaterial verwendete (2R,3R)-4-(2',5'-Dimethoxy-3',4',6'-trimethylphenyl)-2,3-epoxy-2-methylbutanol kann in zu Beispiel 1 analoger Weise hergestellt werden.

## Beispiel 3

237 mg Tosylchlorid und 350 mg (S)-4-(2',5'-Dimethoxy-3',4',6'-trimethylphenyl)-2-methyl-1,2-butandiol wurden in 1 ml Methylenchlorid gelöst. Dann wurden bei 0°C 0,180 ml Pyridin zugetropft und das Gemisch 1 Stunde bei 0°C und dann 16 Stunden bei Raumtemperatur stehen gelassen. Hierauf wurden 1 g Eis und 0,3 ml konzentrierte Salzsäure zugegeben. Dann wurde mit Methylenchlorid extrahiert, der Extrakt getrocknet und eingeengt. Man erhielt 511 mg (95%) (S)-4-(2',5'-Dimethoxy-3',4',6'-trimethylphenyl)-2-methyl-1-toluylsulfonloxy-2-butanol.

$[\alpha]_D^{20}$ +1,2° (c = 2,6% in Chloroform).

177 mg (S)-4-(2',5'-Dimethoxy-3',4',6'-trimethylphenyl)-2-methyl-1-toluylsulfonyloxy-2-butanol

wurden in 1 ml Aethanol gelöst und mit 0,3 ml alkoholischer Kalilauge (1,5N) versetzt. Das Gemisch wurde 10 Minuten bei Raumtemperatur stehen gelassen, dann werden 30 ml Methylenchlorid zugegeben und das Gemisch über Natriumsulfat getrocknet und eingeengt. Man erhielt 105 mg (S)-4-(2',5'-Dimethoxy-3',4',6'-trimethylphenyl)-2-methyl-1,2-epoxybutan. Smp. 47—48°C.

$[\alpha]_D^{20}$ +4,91° (c = 2,2% in Chloroform).

Beispiel 4

5,8 mMol (3R,7R)-3,7,11-Trimethyl-dodecylbromid wurden in 20 ml Aethyläther mit geätztem Magnesium 1/4 Stunde am Rückfluss erhitzt. Dann wurden bei 0°C 1 g (S)-4-(2',5'-Dimethoxy-3',4',6'-trimethylphenyl)-2-methyl-1,2-epoxybutan, 0,9 g Kupfer(I)-2-propylacetylid (bzw. 1,2 g Kupfer(I)-bromid-dimethylsulfid-Komplex) zugesetzt. Die Temperatur des Reaktionsgemisches wurde anschliessend auf Raumtemperatur ansteigen gelassen und das Gemisch wurde über Nacht gerührt. Dann wurden 10 ml Ammoniumchlorid zugegeben und das Gemisch mit Aethyläther extrahiert. Der Extrakt wurde getrocknet, eingeengt und im Kugelrohr destilliert ($Kp_{0,01}$ = 140°C). Man erhält 1,28 g (72%) [bzw. 1,41 g (79%)] (3R,7R,11R)-1-(2',5'-Dimethoxy-3',4',6'-trimethylphenyl)-3,7,11,15-tetramethyl-hexadecan-3-ol als farbloses Oel.

$[\alpha]_D^{20}$ −0,67° (c = 0,9% in Chloroform).

$C_{31}H_{56}O_3$ 476,79) Ber.: C = 78,09; H = 11,84
Gef.: C = 77,92; H = 11,88

Beispiel 5

Zu einer Lösung von 530 mg (1,12 mMol) (3R,7R,11R)-1-(2',5'-Dimethoxy-3',4'-6'-trimethylphenyl)-3,7,11,15-tetramethylhexadecan-3-ol in 50 ml Acetonitril wurden unter Rühren 1,38 g Cer(IV)ammoniumnitrat in 5 ml Wasser zugegeben und diese Mischung wurde während 1 Stunde bei Raumtemperatur gerührt. Das Reaktionsgemisch wurde dreimal mit je 20 ml Methylenchlorid extrahiert, die vereinigten organischen Phasen wurden über Natriumsulfat getrocknet und am Rotationsverdampfer eingedampft. Man erhielt 480 mg (3'R,7'R,11'R)-2-(3'-Hydroxy-3',7',11',15'-tetramethylhexadecan-1'-yl)-3,4,5-trimethyl-1,4-benzochinon.

Das Produkt wurde in 100 ml Methanol gelöst und über Pd/C—10% hydriert. Dann wurden 0,5 ml konzentrierte Salzsäure zugefügt und das Gemisch während 2 Stunden auf 50°C erwärmt. Danach wurde durch Zugabe von festem Natriumhydrogencarbonat neutralisiert und anschliessend filtriert; das Filtrat wurde eingedampft und der Rückstand an Kieselgel mit Toluol/Essigester (2:1) chromatographiert. Man erhielt auf diese Weise 375 mg (90%) 2R,4'R,8'R-α-Tocopherol(d-α-Tocopherol) als schwach gelbliches Oel. Die Enantiomerenreinheit des auf obige Weise erhaltenen d-α-Tocopherols ergab einen Wert von 95%.

**Patentansprüche**

1. Verfahren zur Herstellung von Hydrochinonderivaten sowie gewünschtenfalls von d-α-Tocopherol, dadurch gekennzeichnet, dass man eine Verbindung der allgemeinen Formel

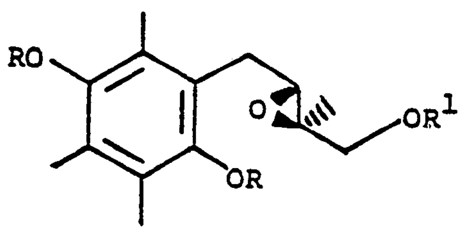

I

worin R eine Aetherschutzgruppe und $R^1$ Wasserstoff oder eine Aetherschutzgruppe darstellen, mit Wasserstoff in Gegenwart von Raney-Nickel oder, falls $R^1$ eine Aetherschutzgruppe darstellt, mit Lithiumaluminiumhydrid, reduziert, dass man die so erhaltene Verbindung der allgemeinen Formel

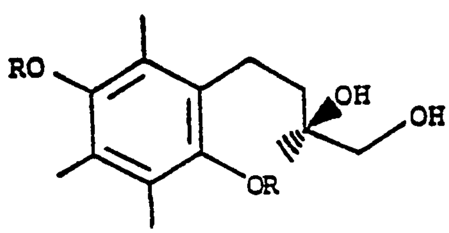

II

worin R die obige Bedeutung hat, in das Epoxid überführt, und dass man das so erhaltene Epoxid der allgemeinen Formel

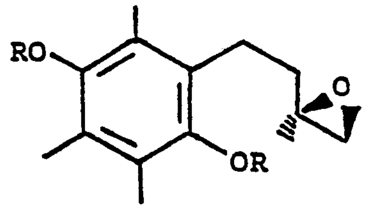

III

worin R die obige Bedeutung hat, mit einer Grignard-Verbindung der allgemeinen Formel

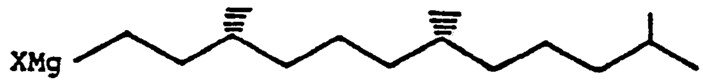

IV

worin X Brom oder Chlor darstellt, umsetzt zur Bildung der entsprechenden Verbindung der allgemeinen Formel

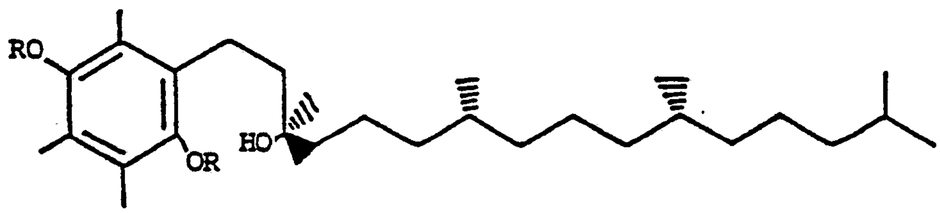

V

worin R die obige Bedeutung hat, in d-α-Tocopherol der Formel welche Verbindung man gewünschtenfalls

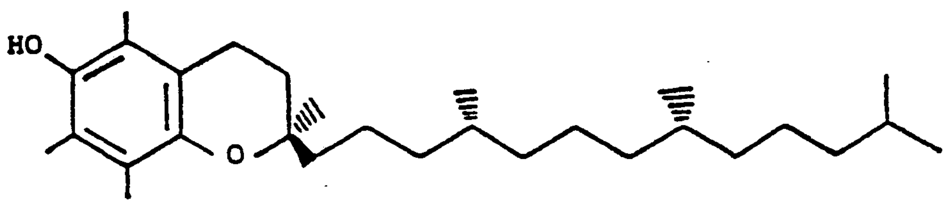

VI

überführt.

2. Verfahren nach Anspruch 1, dadurch gekennzeichnet, dass man die Reduktion einer Verbindung der Formel I mit Raney-Nickel in einem wässrigen Medium durchführt.

3. Verfahren nach Anspruch 1 oder 2, dadurch gekennzeichnet, dass man die Reduktion einer Verbindung der Formel I mit Raney-Nickel im pH-Bereich von etwa 7—10 durchführt.

4. Verfahren nach einem der Ansprüche 1—3, dadurch gekennzeichnet, dass man die Reduktion einer Verbindung der Formel I mit Raney-Nickel bei einer Temperatur von etwa 70°C bis etwa 100°C durchführt.

**Revendications**

1. Procédé de préparation de dérivés de l'hydroquinone et, si on le désire, du d-alpha-tocophérol, caractérisé en ce que l'on reduit un composé de formule générale

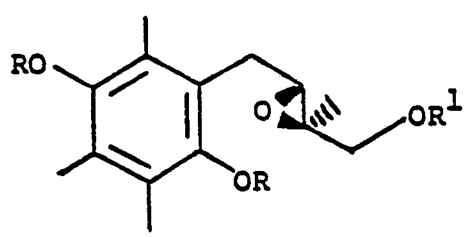

I

dans laquelle R répresente un groupe protecteur éther et $R^1$ l'hydrogène ou un groupe protecteur éther, à l'aide de l'hydogène en présence de nickel de Raney ou bien, lorsque $R^1$ représente un groupe protecteur éther, à l'aide de l'hydrure de lithium et d'aluminium, ce qui donne le composé de formule générale

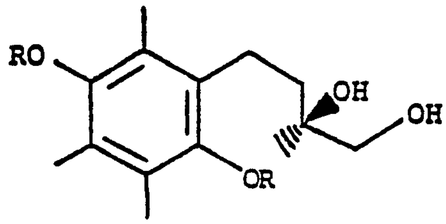

II

dans laquelle R à la signification indiquée ci-dessus, qu'on convertit en l'époxyde, après quoi on fait réagir l'époxyde ainsi obtenu, répondant à la formule générale

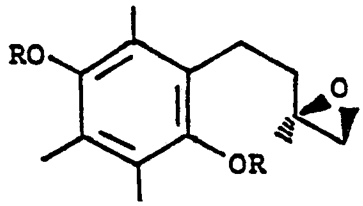

III

dans laquelle R a la signification indiquée ci-dessus, avec un dérivé de Grignard de formule générale

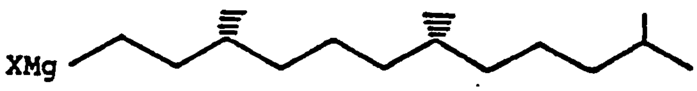

IV

dans laquelle X représente le brome ou le chlore, ce qui donne le composé correspondant de formule générale

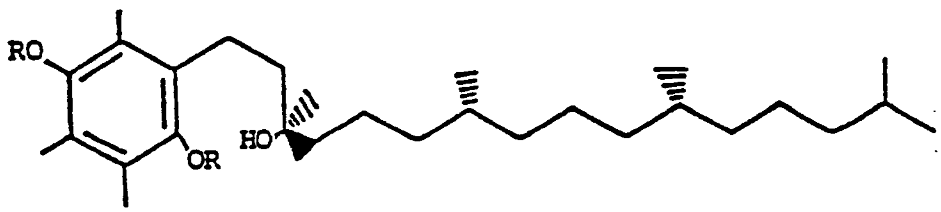

V

dans laquelle R a la signification indiquée ci-dessus qu'on convertit si on le désire en le d-alpha-tocophérol de formule

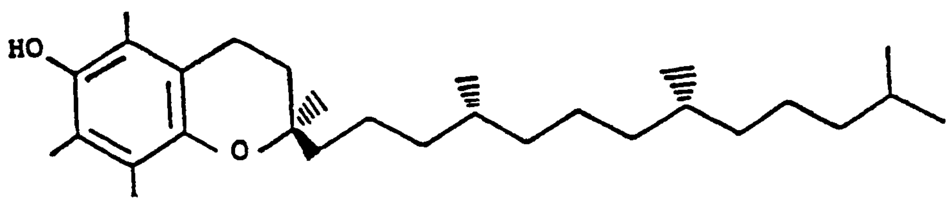

VI

2. Procédé selon la revendication 1, caractérisé en ce que l'on réduit le composé de formule I par le nickel de Raney en milieu aqueux.

3. Procédé selon la revendication 1 ou 2, caractérisé en ce que l'on réduit le composé de formule I par le nickel de Raney à un pH d'environ 7 à 10.

4. Procédé selon l'une des revendications 1 à 3, caractérisé en ce que l'on réduit le composé de formule I par le nickel de Raney à une température d'environ 70 à 100°C.

**Claims**

1. A process for the manufacture of hydroquinone derivatives and, if desired, a d-α-tocopherol, characterized by reducing a compound of the general formula

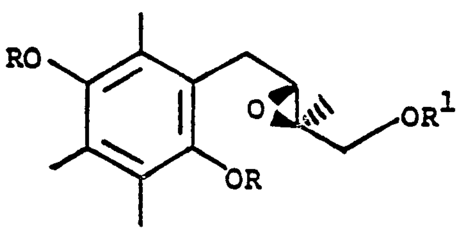

I

wherein R represents an ether protecting group and $R^1$ represents hydrogen or an ether protecting group, with hydrogen in the presence of Raney-nickel or, where $R^1$ represents an ether protecting group, with lithium aluminium hydride, converting the thus-obtained compound of the general formula

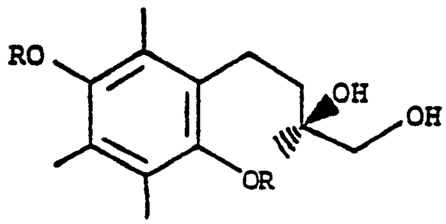

II

wherein R has the above significance, into the epoxide and reacting the thus-obtained epoxide of the general formula

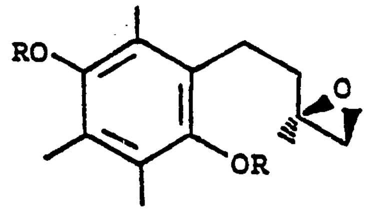

III

wherein R has the above significance, with a Grignard compound of the general formula

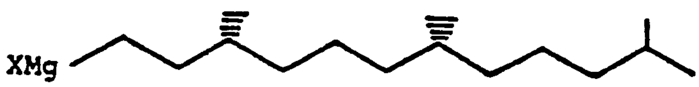

IV

wherein X represents bromine or chlorine, to form the corresponding compound of the general formula

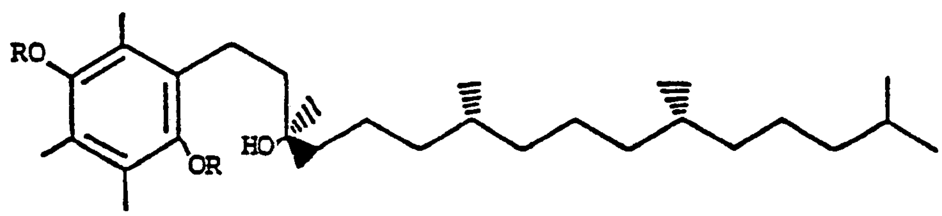

V

wherein R has the above significance which compound is, if desired, converted into d-α-tocopherol of the formula

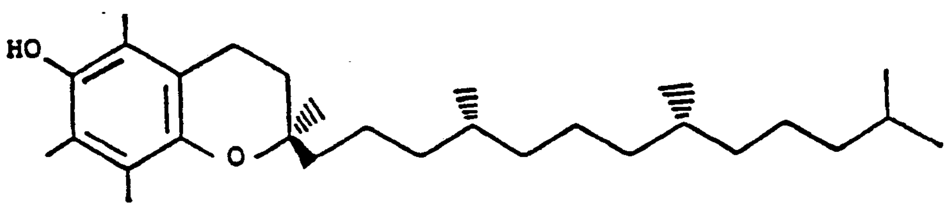

VI

2. A process according to claim 1, characterized in that the reduction of a compound of formula I with Raney-nickel is carried out in an aqueous medium.

3. A process according to claim 1 or 2, characterized in that the reduction of a compound of formula I with Raney-nickel is carried out in the pH-range of about 7—10.

4. A process according to any one of claims 1—3, characterized in that the reduction of a compound of formula I with Raney-nickel is carried out at a temperature of about 70°C to about 100°C.